(19) Europäisches Patentamt
European Patent Office
Office européen des brevets

(11) Veröffentlichungsnummer: **0 246 581 B1**

# (12) EUROPÄISCHE PATENTSCHRIFT

(45) Veröffentlichungstag der Patentschrift: **09.10.91**

(51) Int. Cl.$^5$: **C07D 307/32**

(21) Anmeldenummer: **87107117.1**

(22) Anmeldetag: **16.05.87**

## (54) Verfahren zur Herstellung von alpha-substituierten gamma-Butyrolactonen.

(30) Priorität: **22.05.86 DE 3617177**

(43) Veröffentlichungstag der Anmeldung:
**25.11.87 Patentblatt 87/48**

(45) Bekanntmachung des Hinweises auf die Patenterteilung:
**09.10.91 Patentblatt 91/41**

(84) Benannte Vertragsstaaten:
**CH DE ES FR GB IT LI NL**

(56) Entgegenhaltungen:
**BE-A- 870 555**

**BEILSTEINS HANDBUCH DER ORGANISCHEN CHEMIE, vierte Auflage, drittes und viertes Ergänzungswerk, 1975, Springer Verlag Berlin, Seiten 4229, 4244, 4252**

**METHODEN DER ORGANISCHEN CHEMIE, vierte Auflage, Band VI/2, 1963, Georg Thieme Verlag, Stuttgart, HOUBEN-WEYL:"Sauerstoff-Verbindungen I"**

**REAGENTS FOR ORGANIC SYNTHESIS, Band 3, 1972, Wiley Interscience, New York, US; M. Fieser and L.F. Fieser:"Reagents for Organic Synthesis"**

**CHEMISCHE BERICHTE, Band 101, Heft 4, 1968, Seite 1299-1302, F. NERDEL et al.: "Umwandlung einiger Aldehyde und Ketone in alpha-Halogencarbonsäureester"**

(73) Patentinhaber: **BASF Aktiengesellschaft**
**Carl-Bosch-Strasse 38**
**W-6700 Ludwigshafen(DE)**

(72) Erfinder: **Eckhardt, Heinz, Dr.**
**Ruedigerstrasse 7**
**W-6700 Ludwigshafen(DE)**
Erfinder: **Gramlich, Walter, Dr.**
**Auf der Hoehe 11**
**W-6803 Edingen-Neckarhausen(DE)**
Erfinder: **Best, Walter, Dr.**
**Im Spiess 2**
**W-6714 Weisenheim(DE)**
Erfinder: **Halbritter, Klaus, Dr.**
**Schwarzwaldstrasse 19**
**W-6800 Mannheim 1(DE)**

**EP 0 246 581 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein verbessertes Verfahren zur Herstellung von γ-Butyrolcatonen durch Umsetzen von Acylessigsäurealkylestern mit Alkylenoxiden in Gegenwart von Katalysatoren.

γ-Butyrolactone sind z.T. interressante Riechstoffe, z.T. auch wertvolle Zwischenprodukte für die Synthese zahlreicher heterocyclischer Verbindungen, wie den Pyrrolidonen und Pyrrolidinen.

Die gängigste Methode zur Herstellung von γ-Butyrolactonen ist die von Rothstein (vgl. Compt. Rend. 234 (1952), Seiten 1293 und 1694), bei der entsprechend substituierte Malonsäureester zuerst in das Alkalisalz überführt werden, aus denen dann durch Umsetzung mit einem Alkylenoxid ein γ-Butyrolacton gebildet wird, daß eine zusätzliche Estergruppe enthält, die schließlich verseift und decarboxyliert werden muß. Dieses Verfahren hat aus heutiger Sicht mehrere entscheidende Nachteile. Zum einen ist die Abwasserbelastung durch den Salzanfall infolge der in stöchiometrischen Mengen verwendeten Basen und Säuren recht erheblich. Zum anderen ist der verfahrenstechnische Aufwand - bedingt durch die Notwendigkeit von mehreren Reaktionsschritten, die teilweise in alkalischem und teilweise in saurem Medium stattfinden - recht groß. Außerdem sind die auftretenden Korrosionsprobleme, die verhältnismäßig große Menge an gebildeten Nebenprodukten sowie die hohen Preise für Malonsäureester nachteilig an dem Verfahren. Trotz der genannten deutlichen Nachteile gilt dieses Verfahren als Standardmethode zur Herstellung von γ-Butyrolactonen (vgl. Beilstein, III/IV. Ergänzungswerke, Bd. 17/5, beispielsweise Seiten 4229, 4244 und 4252).

Auch die Herstellung von γ-Butyrolactonen durch Umsetzen von Acylessigsäurealkylestern mit Alkylenoxiden in Gegenwart von Katalysatoren ist im Prinzip schon lange bekannt. So erhält man gemäß Houben-Weyl, "Methoden der organischen Chemie", 4. Auflage, Bd. 6/2, Seite 662 bei der Einwirkung von Ethylenoxid auf Natrium-α-methyl-acetessigsäureethylester in Alkohol das 4-Hydroxy-2-methyl-butansäurelacton (α-Methyl-γ-Butyrolacton) in einer Ausbeute von 50 %.

Bei der Umsetzung von α-Methyl-acetessigsäure-ethylester mit Ethylenoxid in Gegenwart von wasserfreiem Kaliumacetat im geschlossenen Rohr erzielt man Ausbeuten von 70 %, jedoch ist diese Umsetzung ein Ausnahmefall und mit höheren Alkylacetessigestern nicht mehr möglich.

Neben den unbefriedigenden Ausbeuten ist bei diesen Verfahren vor allem der stöchiometrische Verbrauch an Base ein großer Nachteil, so daß versucht wurde, neue Katalysatoren zu finden, bei denen katalytische Mengen ausreichen.

Man fand, daß mit katalytischen Mengen an Natriummethylat keine Umsetzung von α-Alkyl-acetessigsäureestern mit Ethylenoxid stattfand, daß man aber mit Hilfe einer Superbase - Tetrabutylammoniumhexachloroantimonat- zu einem Reaktionsprodukt kommt, das nach Folgeumsetzungen mit Alkali und Säure schließlich ein entsprechendes Lacton liefert. Ausbeuten für diese Reaktion wurden nicht angegeben (vgl. Henkel-Referate 20 (1984), Seiten 61-66, bes. Seiten 65-66).

Es war daher die Aufgabe der Erfindung, das Verfahren zur Herstellung von γ-Butyrolactonen durch Umsetzen von Acylessigsäurealkylestern mit Alkylenoxiden so zu verbessern, daß es einerseits universeller anwendbar ist und andererseits mit geringem Mengen eines möglichst einfach zugänglichen Katalysators durchgeführt werden kann.

Es wurde nun gefunden, daß die Umsetzung von Acetessigsäureestern mit Alkylenoxiden mit Ausbeuten von 90 % der Theorie und mehr erfolgt, wenn man als Katalysatoren ionische Halogenide verwendet. Dieser Befund ist überraschend da Halogenid-Ionen als sehr schwache Basen eingestuft werden und man aufgrund der Literaturergebnisse entweder besonders starke Basen oder Salze mit Komplexen Anionen bevorzugen sollte. Auch die Verwendung des Tetrabutylammonium-kations kann nicht maßgebend für das Gelingen der Reaktion sein, da Alkalihalogenide ebenso gute Ergebnisse liefern wie quartäre Ammoniumhalogenide.

Gegenstand der Erfindung ist dementsprechend ein Verfahren zur Herstellung von γ-Butyrolactonen der allgemeinen Formel I

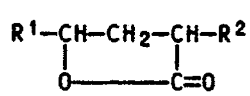

(I),

in der

$R^1$ für Wasserstoff oder eine Alkylgruppe mit 1 bis 4 C-Atomen steht, die durch eine niedere Alkoxygruppe oder eine Acyloxygruppe, vorzugsweise die Acetoxygruppe, substituiert sein kann, steht, und

$R^2$ eine geradkettige oder verzweigte Alkylgruppe mit 1 bis 12 C-Atomen, vorzugsweise 1 bis 4 C-Atomen, die gegenbenenfalls durch funktionelle Gruppen wie eine niedere Alkoxygruppe, eine Acyloxygruppe, eine Amidogruppe, eine Hydroxygruppe, eine Arylgruppe oder Halogenatome substituiert sein kann, oder aber eine Arylgruppe, insbesondere eine Phenylgruppe, bedeuten kann

durch Umsetzen von einem Alkylenoxide der allgemeinen Formel II

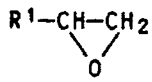

(II)

mit einem Acylessigsäureester der allgemeinen Formel III

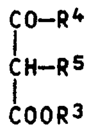

(III).

in der

$R^1$ die oben angegebenen Bedeutung hat,

$R^3$ und $R^4$ für eine verzweigte oder geradkettige Alkylgruppe mit 1 bis 6 C-Atomen, vorzugsweise 1 oder 2 C-Atomen oder eine Arylgruppe, vorzugsweise eine Phenylgruppe stehen und

$R^4$ zusätzlich Wasserstoff bedeuten kann, und

$R^5$ für Wasserstoff oder einen der für $R^2$ genannten Reste steht,

in Gegenwart von Katalysatoren bei erhöhter Temperatur,

das dadurch gekennzeichnet ist, daß man die Umsetzung in Gegenwart von 0,001 bis 0,1 Mol eines Alkalihalogenids, eines Ammoniumhalogenids, vorzugsweise eines quartären Ammoniumhalogenids, eines Phosphoniumhalogenids, eines Alkali-Phosphats oder eines Alkali-Carbonats pro Mol Acylessigester bei Temperaturen von 20 bis 200° C, vorzugsweise 60 bis 150° C und einem Druck von 1 bis 50 bar, vorzugsweise 1 bis 20 bar, durchführt.

Als Katalysatoren können Alkalihalogenide eingesetzt werden, wie LiCl, NaCl, KCl, KF, KBr, NaJ, LiF, NaBr und KJ. Eine weitere Gruppe von geeigneten Katalysatoren stellen Ammoniumhalogenide oder Phosphoniumhalogenide, vorzugsweise quartäre Ammonium- oder Phosphoniumhalogenide dar, wobei bei den bevorzugten Ammonium-Kationen entweder alle vier Substituenten niedere Alkylreste mit 1 bis 6 Kohlenstoff-Atomen sind oder einer oder mehrere der vier Reste eine Benzyl-Gruppe oder eine längere Alkylgruppe mit 6 bis 20 Kohlenstoff-Atomen darstellt. Beispiele sind Tetramethyl-ammoniumchlorid, Tetraethyl-ammoniumbromid, Tetrabutyl-ammonium-chlorid, Benzyl-trimethyl-ammoniumbromid und Methyl-trialkyl-ammonium-chloride, die unter den Namen Adogen 464 der Firma Schering AG oder Aliquat® 336 der Firma General Mills Inc., USA gehandelt werden. Bevorzugte quartäre Phosphonium-halogenide sind z.B. Triphenyl-methyl-phosphoniumchlorid, Triphenyl-ethyl-phosphoniumchlorid, Tributyl-methyl-phosphoniumbromid, Trimethoxy-methyl-phosphoniumbromid und Triethoxy-methyl-phosphoniumbromid. Von den Halogenid-Anionen verwendet man bevorzugt Fluorid, Chlorid, Bromid und lodid. Es ist aber auch möglich andere Anionen zu verwenden, die als Pseudohalogenide bezeichnet werden, wie z.B. Cyanid, Azid, Isocyanat und Rhodanid oder Phosphat und Carbonat, von denen die Phosphate oder Carbonate auch technisches Interesse erlangen könnten.

Als bevorzugte Alkylenoxide der allgemeinen Formel II seien beispielsweise Ethylenoxid, Propylenoxid, Butylenoxid, Acetoxyethylenoxid, Methoxyethylenoxid oder Ethoxyethylenoxid genannt.

Als geeignete Acylessigsäureester der allgemeinen Formel III sind unsubstituierte Acylessigsäureester und in α-Stellung substituierte Acylessigsäureester zu nennen, wobei für die Durchführung der Reaktion die Art des zur Veresterung verwendeten Alkohols keine entscheidende Rolle spielt. Normalerweise werden Ester der niederen Alkohole, d.h. Alkohole mit bis zu 6 C-Atomen, insbesondere Ester des Methanols oder Ethanols verwendet.

Der Rest $R^5$ in den in α-Stellung substituierten Acetessigsäureestern kann ein Alkyl- oder Arylrest sein, der selbst mit weiteren funktionellen Gruppen, wie niederen Alkoxygruppen, niederen Acyloxygruppen, Amidogruppen, Hydroxygruppen, Arylgruppen oder Halogenatomen substituiert sein kann.

Bevorzugte Reste $R^5$ sind Methyl-, Ethyl-, Propyl-, Isopropyl-, Butyl-, Isobutyl-, s-Butyl-, Amyl-, Isoamyl-, 2-Methyl-butyl-, Hexyl-, 2-Ethyl-butyl-, 2-Ethyl-hexyl-, Cyclohexyl-, Heptyl-, 2-Propyl-pentyl-, Octyl-, Nonyl-, Decyl-, Phenyl-, Benzyl-, 2-Hydroxy-ethyl-, 2-Hydroxy-propyl-, 2-Hydroxy-butyl-, Carbomethoxy-methyl- und Carbomethoxy-ethylgruppen.

Der Rest $R^4$ in III kann prinzipiell ebenso breit variiert werden wie der Rest $R^5$. Da er jedoch im Endprodukt I der Umsetzung nicht mehr erscheint, ist es zweckmäßig, an dieser Stelle Substituenten vorzusehen, die einfach zugänglich sind. Das sind z.B. die Phenyl-, die Ethyl- oder die Propylgruppe, insbesondere jedoch die Methylgruppe.

Bei der Umsetzung von in $\alpha$-Stellung substituierten Acetessigsäureestern ist es ganz einfach so, daß der Rest $R^5$ des Acylessigsäureesters in $\alpha$-Stellung des $\gamma$-Butyrolactons der Formel I wieder erscheint, d.h., daß $R^2$ gleich $R^5$ ist. Werden dagegen in $\alpha$-Stellung unsubstituierte Acylessigsäureester verwendet (d.h. $R^5$ = H), so findet im allgemeinen mit beiden in $\alpha$-Stellung stehenden aktivierten Wasserstoffatomen eine Reaktion mit dem Alkylenoxid der Formel II statt. Dies gilt in besonderem Maße für die aktiven niederen Alkylenoxide, wie Ethylenoxid ($R^1$ = H) und Propylenoxid ($R^1$ = $CH_3$). Durch anschließende intramolekulare Umlagerungen bilden sich daher bei der Umsetzung von jeweils 2 Mol eines niederen Alkylenoxids mit 1 Mol eines in $\alpha$-Stellung unsubstituierten Acetessigsäureesters der Formel III $\gamma$-Butyrolactone der allgemeinen Formel I, in der $R^2$ für eine 2-Hydroxy-alkylgruppe oder eine 2-Acetoxy-alkylgruppe steht. Die Bildung dieser $\gamma$-Butyrolactone kann durch folgende Reaktionsgleichung erläutert werden:

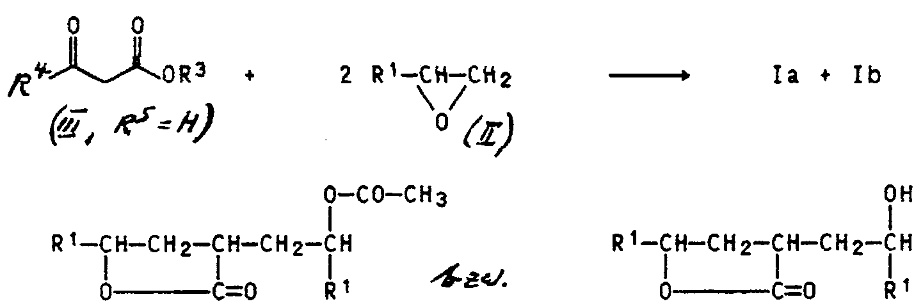


(Ia) (Ib)

Im allgemeinen bilden sich hierbei Gemische der $\gamma$-Butyrolactone der Formeln Ia und Ib ($R^1$ = H, $CH_3$ oder $C_2H_5$). Durch Variation der Reaktionsbedingungen kann man jedoch die bevorzugte Bildung von Ia oder Ib erreichen. So erhält man beispielsweise bei Verwendung von wenig Lösungsmittel bevorzugt die $\alpha$-(2-Acetoxy-alkyl)-verbindung, dagegen in mehr Lösungsmittel bevorzugt die $\alpha$-(2-Hydroxy-alkyl)-verbindung. Der Umsatz zu Ib kann vervollständigt werden durch Behandeln mit geringen Mengen eines Alkalialkoholats.

Die günstigsten Reaktionsbedingungen können für jedes gewünschte $\gamma$-Butyrolacton in einfachen Vorversuchen ermittelt werden.

Gegenstand der Erfindung ist daher auch ein Verfahren wie oben näher beschrieben zur Herstellung von $\gamma$-Butyrolactonen der allgemeinen Formel I, in der $R^1$ für Wasserstoff und $R^2$ für eine 2-Hydroxyethylgruppe oder eine 2-Acetoxyethylgruppe steht, das dadurch gekennzeichnet ist, daß man jeweils 1 bis 3 Mol Ethylenoxid mit 1 Mol eines Acetessigsäureesters der allgemeinen Formel III, in der $R^5$ für Wasserstoff steht und $R^3$ die in Anspruch 1 angegebene Bedeutung hat, umsetzt, sowie ein solches Verfahren zur Herstellung von $\gamma$-Butyrolactonen der allgemeinen Formel I in der $R^1$ für eine Methylgruppe und $R^2$ für eine 2-Hydroxy-1-propylgruppe oder eine 2-Acetoxy-1-propylgruppe steht, das dadurch gekennzeichnet ist, daß man jeweils 1 bis 3 Mol Propylenoxid mit 1 Mol eines Acetessigsäureesters der allgemeinen Formel III, in der $R^5$ für Wasserstoff steht und $R^3$ die in Anspruch 1 angegebene Bedeutung hat, umsetzt.

Die Umsetzungen werden vorzugsweise in einem niederen Alkohol als Lösungsmittel durchgeführt; dabei ist es zum Erreichen eines guten Reaktionsergebnisses nicht notwendig, daß Lösungsmittel und Alkoholkomponente der Estergruppe im Acylessigsäureester III identisch sind. Bevorzugte Lösungsmittel sind Methanol, Ethanol, Propanol, Isopropanol, Butanol und tert.-Butanol.

Die Umsetzungen werden bei Temperaturen zwischen Raumtemperatur und 200 °C, bevorzugt zwischen 60 und 150 °C und zweckmäßig bei erhöhtem Druck von 1 bis 50 bar, vorzugsweise 1 bis 20 bar, durchgeführt. Die Reaktionszeiten liegen zwischen 5 und 20 Stunden, bevorzugt 8 bis 15 Stunden.

In einer bevorzugten Ausführungsform werden Lösungsmittel, Katalysator und Acylessigsäureester III vorgelegt und das Alkylenoxid II bei der Reaktionstemperatur innerhalb von 1 bis 10 Stunden zudosiert. Es ist aber auch möglich, das Alkylenoxid II von Reaktionsbeginn an mit vorzulegen. Nach einer Nachreaktionszeit, die so bemessen wird, daß die oben genannte Gesamtreaktionszeit eingehalten wird, kann das

Reaktionsprodukt auf konventionelle Weise (Filtration, Extraktion, Destillation usw.) isoliert werden.

Die Ausgangsstoffe III und II können im stöchiometrischen Mengenverhältnis oder im Überschuß einer Komponente zur anderen eingesetzt werden. Zweckmäßige Verhältnisse liegen bei 0,5 bis 3 Mol, vorzugsweise 0,8 bis 1,5 Mol Alkylenoxid II je Mol Acetessigsäureester III, wenn keine equimolare Stöchiometrie vorhanden ist. Das Lösungsmittel wird mit einem Anteil von 0 bis 500 % eingesetzt. Die Einsatzmenge des Katalysators beträgt 0,001 bis 0,1 Mol, bevorzugt 0,003 bis 0,05 Mol, je Mol III.

Zum Erreichen einer besonders hohen Produktreinheit kann es, insbesondere bei Verwendung von α-Alkyl-acetessigestern der Formel III mit längeren α-Alkylresten, wie dem α-Hexyl-acetessigsäureethylester, von Vorteil sein, das Reaktionsgemisch nach der erfindungsgemäßen Umsetzung zur Vervollständigung der I-Bildung (ausgehend von Ausgangsverbindungen III mit $R^5 \neq H$) oder der Ib-Bindung (ausgehend von Ausgangsverbindungen III mit $R^5 = H$) noch mit einer geringen Menge einer starken Base zu behandeln. Diese Base muß anschließend durch Behandeln mit einer Mineralsäure wieder neutralisiert werden. Auf diese Weise erhält man auch diese Butyrolactone dann in Ausbeuten von über 90 % in hervorragender Reinheit.

Als starke Basen kommen hierfür in Betracht:
Alkalihydroxide, Alkalialkoholate und tertiäre Amine, insbesondere $NaOCH_3$.

Die verwendete Menge beträgt im allgemeinen 0,1 bis 0,5 Mol pro Mol III. Als Mineralsäure kommen in Betracht: Salzsäure, Schwefelsäure, Phosphorsäure und Salpetersäure.

Mit Hilfe des erfindungsgemäßen Verfahrens können die als Riechstoffe bzw. Zwischenprodukte für die Synthese von Heterocyclen begehrten γ-Butyrolactone der allgemeinen Formel I durch umsetzen der Acylessigsäureester III mit den Alkylenoxiden II auf einfache und billige Weise, ohne große Abwasserprobleme aufzuwerfen, in sehr guten Ausbeuten hergestellt werden.

Die nachfolgenden Beispiele sollen das Verfahren erläutern.

<u>Beispiele 1 bis 8</u>

In einem 300 ml-Autoklaven wurde eine Mischung bestehend aus den in der folgenden Tabelle angegebenen α-Alkyl-acetessigestern der Formel III in der dort angegebenen Menge, sowie der gleichen Gew.-Menge des angegebenen Lösungsmittels, jeweils 1,5 Gew.%, bezogen auf eingesetztes III, an Tetramethyl-ammoniumchlorid und der dort angegebenen Menge an Ethylenoxid 12 Stunden (h) lang auf 100 °C erhitzt.

Destillation der Reaktorausträge ergab jeweils die in der Tabelle angegebenen Ausbeuten an dem entsprechenden α-Alkyl-butyrolacton der Formel I

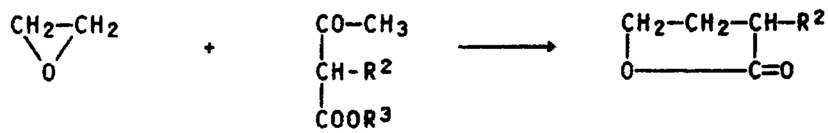

| Beispiel | Acetessigsäurealkylester III $R^2$ | $R^3$ | Menge [g] | Lösungsmittel | Ethylenoxid II Menge [g] | I-Ausbeute [g] | [%] |
|---|---|---|---|---|---|---|---|
| 1 | $-CH_3$ | $-CH_3$ | 78 | Methanol | 31,7 | 55,2 | 92 |
| 2 | $-C_2H_5$ | $-C_3H_7$ | 120 | Propanol | 37,0 | 69,4 | 87 |
| 3 | $-C_4H_9$ | $-C_2H_5$ | 86 | Ethanol | 22,0 | 59,7 | 91 |
| 4 | $-CH_2-\overset{CH_3}{\overset{\vert}{C}}H-CH_2-CH_3$ | $-C_2H_5$ | 89 | Ethanol | 23,5 | 61,1 | 88 |
| 5 | $-C_6H_{13}$ | $-C_2H_5$ | 64 | Ethanol | 15,8 | 47,4 | 93 |
| 6 | $-C_6H_{13}$ | $-CH_3$ | 100 | Methanol | 26,4 | 76,5 | 90 |
| 7 | $-CH_2-\overset{C_2H_5}{\overset{\vert}{C}}H-CH_2-CH_3$ | $-C_2H_5$ | 64 | Ethanol | 15,8 | 46,4 | 91 |
| 8 | 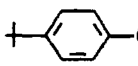 $-CH_2-$ | $-C_2H_5$ | 100 | Ethanol | 19,1 | 75,2 | 90 |

## Beispiel 9

In einem 20-1-Autoklaven wurde eine Mischung bestehend aus 8,56 kg (40 mol) α-Hexyl-acetessigsäureethylester, 0,95 kg Methanol und 80 g (0,38 mol) Triethyl-benzyl-ammoniumchlorid erhitzt. Bei 100°C wurden innerhalb von 4 h 2,11 kg (= 52,8 Mol) Ethylenoxid zudosiert, danach der Druck mit Stickstoff auf 20 bar erhöht und das Reaktionsgemisch noch 10 h bei 100°C nachreagieren lassen.

Der Reaktionsaustrag wurde dann zwecks Erhalt eines besonders reinen Produktes zur Vervollständigung der I-Bildung bei Normaldruck mit 500 g (9,26 mol) Natriummethylat in Form einer 30 %igen methanolischen Lösung versetzt und dann noch 4 h auf 65°C erhitzt. Danach wurde das Reaktionsgemisch eingeengt und 3 h bei 100°C mit 3 1 2 n Schwefelsäure behandelt. Durch destillative Aufarbeitung der organischen Phase erhielt man 6,33 kg 3-Hexyl-butyrolacton in einer Reinheit von 99,9 %. Die Ausbeute betrug 93 % der Theorie.

## Beispiel 10

Eine Mischung aus 107 g α-Hexyl-acetessigsäureethylester, 107 g Ethanol, 34 g Propylenoxid und 1,5 g Tetramethyl-ammoniumchlorid wurde in einem Autoklaven 10 h lang auf 120°C erhitzt. Der Reaktionsaustrag wurde mit 5 g Natriumethylat noch 5 h auf 65°C erhitzt und dann destilliert. Man erhielt 84,6 g 3-Hexyl-5-methyl-butyrolacton. Die Ausbeute betrug 92 % der Theorie.

## Beispiel 11

Eine Mischung aus 58 g Acetessigsäuremethylester, 44 g Ethylenoxid, 3 g NaCl und 70 ml Methanol wurde in einem Autoklaven unter Eigendruck 12 h lang auf 80°C erhitzt. Anschließend wurde das Methanol abdestilliert, der Rückstand mit Wasser aufgenommen, mit wenig Chloroform gewaschen und destilliert. Man erhielt 52,6 g 2-(2-Hydroxy-ethyl)-γ-butyrolacton. Das entspricht einer Ausbeute von 81 % der Theorie.

Darüber hinaus sind noch 9,3 g (entsprechend 8 % der Theorie) an 2-(2-Hydroxy-ethyl)-γ-butyrolacton in dem Waschchloroform enthalten, was aufgearbeitet werden könnte.

## Beispiel 12

Man arbeitete wie in Beispiel 11 beschrieben, verwendete jedoch anstelle von 3 g NaCl 10 g $K_2CO_3$. Es wurden 54 g 2-(2-Hydroxy-ethyl)-γ-butyrolacton erhalten. Das entspricht einer Ausbeute von 83 % der Theorie.

Beispiel 13

Man arbeitete wie in Beispiel 11 beschrieben, verwendete jedoch anstelle von 3 g NaCl 10 g $Na_3PO_4 \bullet 12H_2O$.. Man erhielt 21,7 g (entsprechend 34 % Ausbeute) 2-(2-Hyroxy-ethyl)-γ-butyrolacton und 50,7 g (entsprechend 59 % Ausbeute) an 2-(2-Acetoxyethyl)-γ-butyrolacton vom Siedepunkt Kp = 100 bis 105 °C bei 0,3 mbar.

Beispiel 14

58 g (0,5 mol) Acetessigsäuremethylester, 44 g Ethylenoxid, 3 g NaCl und 30 g Methanol wurden im Autoklaven unter Eigendruck 12 h lang auf 80 °C erhitzt. Anschließend destillierte man das Methanol ab, nahm den Rückstand in Chloroform auf, wusch mit wenig Wasser und destillierte. Man erhielt 65,3 g α-(2-Acetoxy-ethyl)-γ-butyrolacton vom Siedepunkt Kp = 100 bis 105 °C bei 0,3 bar. Das entspricht einer Ausbeute von 76 % der Theorie. In der wäßrigen Phase sind noch 8,5 g α-(2-Hydroxy-ethyl)-γ-butyrolacton (entsprechend 13 % der Theorie) enthalten.

**Patentansprüche**

**1.** Verfahren zur Herstellung von α-substituierten γ-Butyrolactonen der allgemeinen Formel I

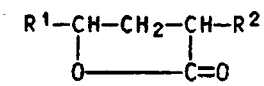

(I),

in der

- $R^1$ für Wasserstoff oder eine Alkylgruppe mit 1 bis 4 C-Atomen steht, die durch eine niedere Alkoxygruppe oder eine niedere Acyloxygruppe substituiert sein kann und
- $R^2$ eine geradkettige oder verzweigte Alkylgruppe mit 1 bis 12 C-Atomen, die gegebenenfalls durch funktionelle Gruppe wie eine niedere Alkoxygruppe, eine Acyloxygruppe, eine Amidogruppe, eine Hydroxygruppe, eine Arylgruppe oder Halogenatome substituiert sein kann oder aber eine Arylgruppe bedeuten kann,

durch Umsetzen von einem Alkylenoxid der allgemeinen Formel II

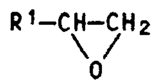

(II)

mit einem Acylessigsäureester der allgemeinen Formel III

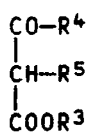

(III).

der

- $R^1$ die oben angegebene Bedeutung hat,
- $R^3$ und $R^4$ für eine verzweigte oder geradkettige Alkylgruppe mit 1 bis 6 C-Atomen oder eine Arylgruppe stehen,
- $R^4$ zusätzlich Wasserstoff bedeuten kann, und
- $R^5$ für Wasserstoff oder einen der für $R^2$ genannten Reste steht,

in Gegenwart von Katalysatoren bei erhöhter Temperatur,

dadurch gekennzeichnet, daß man die Umsetzung in Gegenwart von 0,001 bis 0,1 Mol eines Alkalihalogenids, eines Ammoniumhalogenids, vorzugsweise eines quartären Ammoniumhalogenids, eines Phosphoniumhalogenids, eines Alkali-Phosphats, oder eines Alkali-Carbonats pro Mol Acylessigester bei Temperaturen von 20 bis 200 °C und einem Druck von 1 bis 50 bar durchführt.

2. Verfahren zur Herstellung von γ-Butyrolactonen der allgemeinen Formel I gemaß Anspruch 1, dadurch gekennzeichnet, daß man die Umsetzung bei Temperaturen von 60 bis 150 °C durchführt.

3. Verfahren zur Herstellung von γ-Butyrolactonen der allgemeinen Formel I gemäß Anspruch 1, dadurch gekennzeichnet, daß man die Umsetzung bei einem Druck von 1 bis 20 bar durchführt.

4. Verfahren zur Herstellung von γ-Butyrolactonen der allgemeinen Formel I gemäß Anspruch 1, dadurch gekennzeichnet, daß man das Reaktionsgemisch anschließend noch 1 bis 10 Stunden bei Temperaturen von 20 bis 150 °C mit 0,02 bis 0,5 Mol einer starken Base behandelt und diese dann mit einer Mineralsäure neutralisiert.

5. Verfahren gemäß Anspruch 1 zur Herstellung von γ-Butyrolactonen der allgemeinen Formel I, in der $R^1$ für Wasserstoff und $R^2$ für eine 2-Hydroxy-ethylgruppe oder eine 2-Acetoxyethylgruppe steht, dadurch gekennzeichnet, daß man jeweils 1 bis 3 Mol Ethylenoxid mit 1 Mol eines Acetessigsäureesters der allgemeinen Formel III, in der $R^5$ für Wasserstoff steht und $R^3$ die in Anspruch 1 angegebene Bedeutung hat, umsetzt.

6. Verfahren gemäß Anspruch 1 zur Herstellung von γ-Butyrolactonen der allgemeinen Formel I, in der $R^1$ für eine Methylgruppe und $R^2$ für eine 2-Hydroxy-1-propylgruppe oder eine 2-Acetoxy-1-propylgruppe steht, dadurch gekennzeichnet, daß man jeweils 1 bis 3 Mol Propylenoxid mit 1 Mol eines Acetessigsäureesters der allgemeinen Formel III, in der $R^5$ für Wasserstoff steht und $R^3$ die in Anspruch 1 angegebene Bedeutung hat, umsetzt.

## Claims

1. A process for the preparation of an α-substituted γ-butyrolactone of the formula I

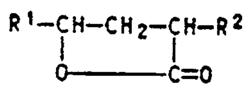

I

where $R^1$ is hydrogen or alkyl of 1 to 4 carbon atoms which may be substituted by lower alkoxy or lower acyloxy and $R^2$ is straight-chain or branched alkyl of 1 to 12 carbon atoms which is unsubstituted or substituted by functional groups such as lower alkoxy, acyloxy, amido, hydroxyl, aryl or halogen, or is aryl, by reacting an alkylene oxide of the formula II

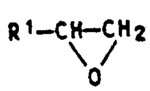

II

with an acylacetate of the formula III

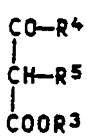

III

where $R^1$ has the above meanings, $R^3$ and $R^4$ are each branched or straight-chain alkyl of 1 to 6 carbon atoms or aryl, $R^4$ may furthermore be hydrogen and $R^5$ is hydrogen or one of the radicals stated for $R^2$, in the presence of a catalyst at elevated temperatures, wherein the reaction is carried out in the presence of from 0.001 to 0.1 mole of an alkali metal halide, of an ammonium halide, preferably a quaternary ammonium halide, of a phosphonium halide, of an alkali metal phosphate or of an alkali

metal carbonate per mole of acylacetate at from 20 to 200° C and under from 1 to 50 bar.

**2.** A process for the preparation of a γ-butyrolactone of the formula I as claimed in claim 1, wherein the reaction is carried out at from 60 to 150° C.

**3.** A process for the preparation of a γ-butyrolactone of the formula I as claimed in claim 1, wherein the reaction is carried out under from 1 to 20 bar.

**4.** A process for the preparation of a γ-butyrolactone of the formula I as claimed in claim 1, wherein the reaction mixture is then treated for a further 1-10 hours at from 20 to 150° C with from 0.02 to 0.5 mole of a strong base, which is then neutralized with a mineral acid.

**5.** A process as claimed in claim 1 for the preparation of a γ-butyrolactone of the formula I where $R^1$ is hydrogen and $R^2$ is 2-hydroxyethyl or 2-acetoxyethyl, wherein from 1 to 3 moles of ethylene oxide are reacted with 1 mole of an acetoacetate of the formula III where $R^5$ is hydrogen and $R^3$ has the meanings stated in claim 1.

**6.** A process as claimed in claim 1 for the preparation of a γ-butyrolactone of the formula I where $R^1$ is methyl and $R^2$ is 2-hydroxyprop-1-yl or 2-acetoxyprop-1-yl, wherein from 1 to 3 moles of propylene oxide are reaoted with 1 mole of an acetoacetate of the formula III where $R^5$ is hydrogen and $R^3$ has the meanings stated in claim 1.

## Revendications

**1.** Procédé de préparation de γ-butyrolactones α-substituees de formule générale I

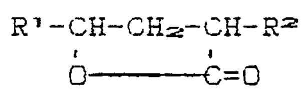

(I)

dans laquelle

$R^1$ est mis pour un atome d'hydrogène ou pour un groupement alkyle à 1-4 atomes de carbone qui peut être substitué par un groupement alcoxy inférieur ou par un groupement acyloxy inférieur et

$R^2$ peut représenter un groupement alkyle à chaîne droite ou ramifiée à 1-12 atomes de carbone, qui peut être éventuellement substitué par des groupements fonctionnels tels qu'un groupement alcoxy inférieur, un groupement acyloxy, un groupement amido, un groupement hydroxy, un groupement aryle ou des atomes d'halogène, ou il peut représenter un groupement aryle.

par réaction d'un oxyde d'alkylène de formule générale II

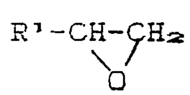

(II)

avec un ester d'acide acylacétique de formule générale III

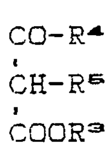

(III)

où

$R^1$ a la signification donnée ci-dessus,

$R^3$ et $R^4$ sont mis chacun pour un groupement alkyle à chaîne droite ou ramifiée à 1-6 atomes de carbone ou pour un groupement aryle,
$R^4$ peut en outre représenter un atome d'hydrogène, et
$R^5$ est mis pour un atome d'hydrogène ou pour l'un des rentes spécifiés à propos de $R^2$,

en présence de catalyseurs à température élevée, caractérisé en ce qu'on conduit la réaction en présence de 0,001 a 0,1 mole d'un halogénure alcalin, d'un halogénure d'ammonium, de préférence d'un halogénure d'ammonium quaternaire, d'un halogénure de phosphonium, d'un phosphate alcalin ou d'un carbonate alcalin par mole d'ester acylacétique, à des températures de 20 à 200 °C et sous une pression de 1 à 50 bar.

**2.** Procédé de préparation de γ-butyrolactones de formule générale I selon la revendication 1, caractérisé en ce qu'on conduit la réaction à des températures de 60 à 150 °C.

**3.** Procédé de préparation de γ-butyrolactones de formule générale I selon la revendication 1, caractérisé en ce qu'on conduit la réaction sous une pression de 1 à 20 bar.

**4.** Procédé de préparation de γ-butyrolactones de formule générale I selon la revendication 1, caractérisé en ce qu'on traite ensuite le mélange réactionnel, pendant 1 a 10 h encore à des températures de 20 à 150 °C, par 0,02 à 0,5 mole d'une base forte, puis on neutralise celle-ci par un acide minéral.

**5.** Procédé selon la revendication 1 pour la préparation de γ-butyrolactones de formule générale I, dans laquelle $R^1$ est mis pour un atome d'hydrogène et $R^2$pour un groupement 2-hydroxyéthyle ou un groupement 2-acetoxyéthyle, caractérisé en ce qu'on fait réagir chaque fois 1 à 3 moles d'oxyde d'éthylène avec 1 mole d'un ester d'acide acétylacétique de formule générale III, dans laquelle $R^5$ est mis pour un atome d'hydrogène et $R^3$ a la signification indiquée dans la revendication 1.

**6.** Procédé selon la revendication 1 pour la préparation de γ-butyrolactones de formule générale I, dans laquelle $R^1$ est mis pour un groupement méthyle et $R^2$ pour un groupement 2-hydroxy-1-propyle ou un groupement 2-acétoxy-1-propyle, caractérisé en ce qu'on fait réagir chaque fois 1 à 3 moles d'oxyde de propylène avec 1 mole d'un ester d'acide acétylacétique de formule générale III, dans laquelle $R^5$ est mis pour un atome d'hydrogène et $R^3$ a la signification indiquée dans la revendication 1.